# EUROPÄISCHE PATENTSCHRIFT

(11) **EP 0 682 924 B1**
(45) Veröffentlichungstag und Bekanntmachung des Hinweises auf die Patenterteilung: **13.10.1999**
(21) Anmeldenummer: 95101952.0
(22) Anmeldetag: 14.02.1995
(51) Int. Cl.: A61F 2/36

(54) **Femurprothese**
Femoral prosthesis
Prothèse fémorale

(30) Priorität: 19.04.1994 CH 117494
(43) Veröffentlichungstag der Anmeldung: 22.11.1995
(73) Patentinhaber: Hermann, Werner, CH-6312 Steinhausen (CH)
(72) Erfinder: Hermann, Werner, CH-6312 Steinhausen (CH)
(74) Vertreter: OK pat AG

(56) Entgegenhaltungen:
- EP-A- 0 209 516
- EP-A- 0 366 945
- EP-A- 0 378 044
- WO-A-83/02555
- DE-A- 3 811 207
- FR-A- 2 315 902
- US-A- 3 064 645

## Beschreibung

Die Erfindung betrifft eine Femurprothese nach dem Oberbegriff des Anspruchs 1.

Aus der **EP-A-0 209 516** ist eine Femurprothese der eingangs genannten Art bekannt. Diese weist Längsrippen auf, welche alle vom proximalen Schaftende ausgehen aber auf verschiedenen Höhen enden. Nicht nur der Schaftkern und die fiktive Hüllkurve an den Schaft sondern auch die einzelnen Rippen selbst verjüngen sich zum distalen Ende hin und bilden dadurch eine Art Keil. Der Schaft wird also beim Einsetzen in den Knochen gewissermassen in diesen eingekeilt. Dabei besteht die Gefahr, dass der Knochen beim Eintreiben des Schaftes durch die sich nach oben im Querschnitt vergrössernden Längsrippen gesprengt wird.

Zwar ist aus der **EP-A-0 378 044** eine Femurprothese bekannt, deren Längsrippen zum Teil abgestuft sind. Der Schaff weist einen oberen, flächigen, sogenannt blattförmigen Bereich und einen0 daran anschliessenden unteren Schaffte auf, der sich zum distalen Ende hin von einer ovalen Form zu einer im wesentlichen runden Form verjüngt. Der blattförmige Bereich besitzt erste Rippen, über welche die Prothese nur an vier Punkten in der harten kortikalen Schale des Knochens abgestützt, sowie zweite Rippen, welche sich im unteren Bereich des Schaftes fortsetzen und dazu bestimmt sind, in der weichen Spongiosa angebracht zu werden. Der Nachteil dieser Prothese ist es, dass sie nur elastisch aber nicht fix im Knochen verankert werden kann.

Eine weitere Femurprothese ist aus der **EP-A-0 366 945** bekannt.

Es handelt sich dort um einen blattförmigen Schaft mit dorsalen und ventralen Breitseiten sowie medialen und lateralen Schmalseiten. An den Breitseiten und ggf. an der lateralen Schmalseite sind mehrere Längsnuten vorhanden, die proximal (nach oben) offen und zur Aufnahme von einzeln einzusetzenden Längsrippen bestimmt sind. Um die Prothese im Oberschenkelknochen zu verankern, muss vom proximalen, offenen Knochenende her um den oberen Bereich des bereits eingesetzten Schaftblattes herum eine Höhlung aus dem Knochen herausmodelliert werden. Anschliessend werden die genannten Rippen in die Längsnuten eingeführt, wobei Rippensätze mit unterschiedlichen Rippenhöhen verfügbar sein müssen und auch die Wand der Knochenhöhlung gegebenenfalls nachbearbeitet werden muss. Schliesslich müssen die Rippen gegen Längsverschiebung in Ihren Nuten mittels weiterer Einzelteile gesichert werden.

Die beschriebene Prothesenkonstruktion ist ausserordentlich kompliziert und wegen der erforderlichen Präzision sehr anspruchsvoll und entsprechend teuer in der Herstellung. Insbesondere erfordert aber die Implantation eine beträchtliche Geschicklichkeit des Chirurgen und ist sehr zeitraubend, wodurch sich die Dauer des chirurgischen Eingriffs erheblich verlängert. Besonders nachteilig ist auch, dass eine relativ weite Implantationshöhlung benötigt wird und entsprechend viel Knochensubstanz abgetragen werden muss. Sodann vermögen die als Einzelteile eingesetzten Längsrippen das Schaftblatt im Röhrenknochen zwar seitlich abzustützen, können aber kaum erhebliche Torsionskräfte übertragen, was ebenso wichtig wäre.

Aufgabe der Erfindung ist es, eine einfach herstell- und implantierbare Femurprothese der eingangs genannten Art zu schaffen, welche so gestaltet ist, dass sie ohne Gefahr für den Knochen eingetrieben und fix verankert werden kann.

Diese Aufgabe wird erfindungsgemäss durch die Merkmale des kennzeichnenden Teils des Anspruchs 1 gelöst. Vorteilhafte Weiterbildungen der erfindungsgemässen Femurprothese werden durch die abhängigen Ansprüche definiert.

Mit der einstückigen Herstellung werden Fertigung und Lagerhaltung der Prothese erheblich vereinfacht. Vor allem aber gestaltet sich die chirurgische Knochenvorbereitung wie auch die Implantation selbst wesentlich einfacher, schneller und schonender, wobei nicht zuletzt eine sichere Verankerung im Femur erreicht wird, indem die Längsrippen nunmehr auch Torsionsbelastungen übertragen können. Dabei wird dank dem achsparallelen, d.h. "nicht konischen" Rippenprofil eine gefährliche Keilwirkung auf den Röhrenknochen beim Eintreiben des Schaftes weitgehend vermieden. stehend wird der Erfindungsgegenstand anhand von Ausführungsbeispielen in Verbindung mit der Zeichnung näher beschrieben.
- Fig. 1: ist die Seitenansicht einer beispielsweisen erfindungsgemässen Femurprothese,
- Fig. 2: ist eine perspektivische Ansicht des Schaftes der Prothese nach Fig. 1,
- Fig. 3a: zeigt die zugehörige Stirnansicht des Schaftes in einem grösseren Massstab,

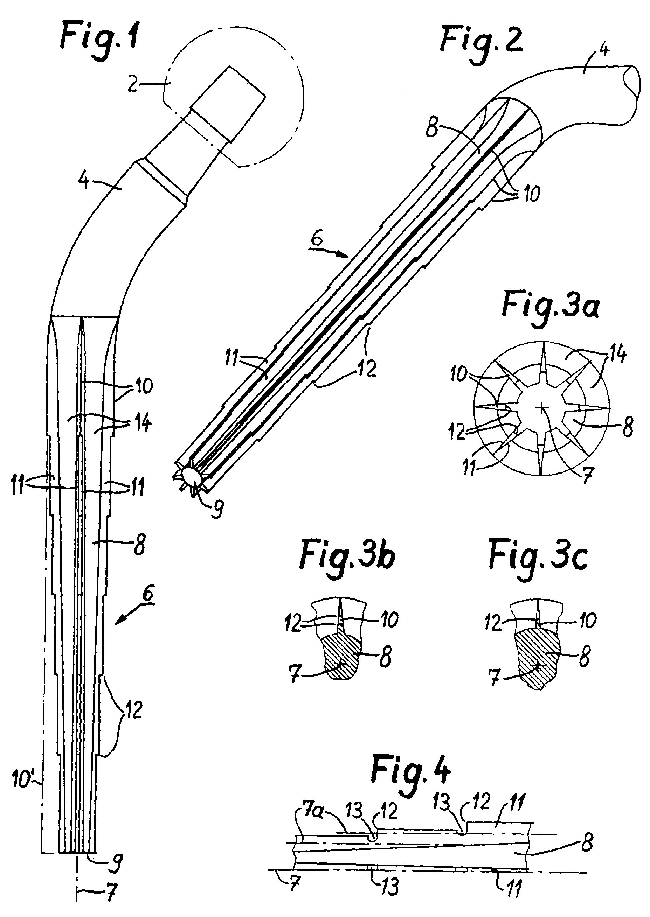

- Fig. 3a: und 3b sind entsprechende Teil-Querschnitte durch den Schaft in zunehmendem Abstand vom distalen Ende des Schaftes, und
- Fig. 4: ist eine hälftige Seitenansicht eines Schaftabschnittes gemäss einer Ausführungsvariante.

Die dargestellte Femurprothese ist einstückig aus Rundmaterial hergestellt und bestehht generell aus einem Schaft 6 mit Längsrippen 10 und einem zum Schaft abgewinkelten Hals 4. Der letztere weist am Ende einen Konuszapfen auf, welcher in an sich bekannter Weise die strichpunktiert angedeutete Gelenkkugel 2 aufnimmt.

Der Schaft 6 wird bei der Hüftgelenks-Operation in Längsrichtung mit dem distalen Ende 9 voran in eine vorbereitete Höhlung des Oberschenkelknochens (Femur, nicht dargestellt) eingetrieben. Der volle Kern 8 des Schaftes 6 ist gegen das distale Ende 9 hin konisch verjüngt. Anstelle des mit kreisförmigem Querschnitt dargestellten Schaftkerns 8 könnte der Schaftkern auch ovalkonisch ausgebildet sein.

Der Schaft 6 ist ferner mit radial vom Schaftkern 8 abstehenden Längsrippen 10 versehen; vorzugsweise sind vier bis acht regelmässig über den Umfang verteilte Längsrippen vorgesehen (in den Fig. 1 und 4 sind zwecks besserer Uebersicht nur vier Rippen dargestellt anstelle von acht Rippen wie in Fig. 2 und 3a-3c).

Die Längsrippen 10 werden zweckmässigerweise durch Einfräsen von zwischenliegenden Längsnuten 14 aus dem vollen Rundmaterial hergestellt, so dass sie mit dem Schaftkern 8 einstückig verbunden sind. Wesentlich ist, dass die die Rippen 10 begrenzenden Seitenflanken 11 durch Mantellinien 7a (Fig. 4) gebildet sind, die zur Längsachse 7 des Schaftes 6 parallel verlaufen. In der Zeichnung ist dies ausser aus Fig. 4 daraus ersichtlich, dass die Rippenquerschnitte nach Fig. 3b und 3c - bei zunehmendem Abstand vom distalen Ende 9 und verminderter Rippenhöhe entsprechend dem zunehmenden Durchmesser des konischen Schaftkerns 8 - immer Teilquerschnitte desselben spitzwinkligen Dreiecks sind, welches in der Stirnansicht nach Fig. 3a erscheint; beim dargestellten Dreiecks-Querschnitt verändert sich dabei auch die Breite der Rippenbasis am Uebergang zum Schaftkern 8 entsprechend der Konizität des Letzteren. Der Rippenquerschnitt muss jedoch nicht dreieckig sein, sondern könnte auch z.B. durch ein "schlankes" Rechteck oder Trapez bestimmt sein.

Beim erwähnten Eintreiben des Schaftes 6 in den röhrenförmigen Oberschenkelknochen "graben" sich die Längsrippen 10 zunehmend in das Knochenmaterial ein, welches die vorbereitete Knochen-Längsbohrung umgibt. Dabei wird dank dem vorstehend beschriebenen Verlauf der Seitenflanken 11 der Rippen (parallele Mantellinien) jegliche mechanische Spannung in Umfangsrichtung des Knochens vermieden, d.h. der Knochen wird nur in Längsrichtung belastet und es besteht keine Tendenz, diesen in gefährlicher Weise aufzusprengen oder zu spalten. Auch die Reibung der Seitenflanken 11 am Knochen beim Vortrieb des Schaftes 6 ist relativ gering.

Das Eintreiben wird indessen noch zusätzlich erleichtert und die Längsführung des Schaftes 6 zu Beginn des Eintreibens verbessert, indem die Längsrippen 10 wie dargestellt in ihrer Höhe abgestuft sind, wobei mehrere über die Länge der Rippen verteilte Schneidkanten 12 gebildet werden. Das Stufenverhältnis (Stufenhöhe zu Stufenlänge) wird dabei zweckmässig etwa gleich dem Konusverhältnis des Schaftkerns 8 gewählt. Wie bei der Variante nach Fig. 4 dargestellt, können an die Kanten 12 anschliessende Spannuten 13 zur Aufnahme des angeschnittenen Knochenmaterials vorgesehen werden.

Die beschriebene, erfindungsgemässe Femurprothese lässt sich relativ einfach herstellen. Sie ist vor allem in der Anwendung vorteilhaft, indem die Vorbereitung des Femurs durch den Chirurgen sehr rasch und einfach von statten geht und nur wenig Knochenmaterial abgetragen werden muss. Dabei ergibt sich auch eine besonders sichere, dauerhafte Verankerung des Prothesenschaftes im Röhrenknochen, die ausser Biegebeanspruchungen auch Torsionskräfte einwandfrei in den Knochen einzuleiten vermag.

## Patentansprüche

1. Femurprothese mit einem zum Eintreiben in einen Oberschenkelknochen bestimmten Schaft (**6**), der zum distalen Ende (**9**) hin verjüngt ist und dessen Hüllfläche runde Querschnitte besitzt, welcher Schaff einen konischen Schaftkern (**8**) sowie seitlich vom Schaftkern (**8**) abstehende Längsrippen (**10**) umfasst, welche mit dem Schaftkern (**8**) einstückig verbunden sind,
**dadurch gekennzeichnet,**
- dass die Längsrippen (**10**) in ihrer radialen Höhe mehrfach abgestuft sind und an den dabei gebildeten Stufen Schneidkanten (**12**) aufweisen und
- dass die Begrenzungsflächen (**11**) der Längsrippen (**10**) durch Mantellinien (**7a**) gebildet sind, die parallel zur Längsachse (**7**) des Schaftes (**4**) verlaufen,
- wobei jede der vom Schaftkern (**8**) ausgehenden Flanken der Längsrippen (**10**) über ihre gesamte Länge den gleichen Abstand von der Längsachse (**7**) aufweist, und
- jeder eine äussere Begrenzung einer der Längsrippen (**10**) bildende Teil zwischen zwei benachbarten Stufen einen konstanten Abstand von der Längsachse (**7**) aufweist.

2. Femurprothese nach Anspruch 1,
**dadurch gekennzeichnet,**
dass die Längsrippen (**10**) im Bereich der Stufen mit Spannuten (**13**) versehen sind.

3. Femurprothese nach einem der vorangehenden Ansprüche,
**dadurch gekennzeichnet,**
dass die Längsrippen (**10**) regelmässig über den Umfang des Schaftkernes (**8**) verteilt angeordnet sind.

4. Femurprothese nach einem der vorangehenden Ansprüche,
**dadurch gekennzeichnet,**
dass der Querschnitt jeder Längsrippe (**10**) am proximalen Ende ein spitzwinkliges Dreieck, ein Trapez oder ein Rechteck mit einer zwei Punkte des Schaftkernes verbindenden Geraden als Grundlinie und den von diesen Punkten ausgehenden Flanken als Seitenlinien ist.

5. Femurprothese nach einem der vorangehenden Ansprüche,
**dadurch gekennzeichnet,**
dass sie aus Rundmaterial hergestellt ist, wobei die Längsrippen (**10**) durch zwischenliegende, herausgearbeitete Nuten (**14**) gebildet sind.

## Claims

1. A femoral prosthesis having a shank (6) which is intended to be driven into a femur and which is tapered towards its distal end (9), and the external surface of which comprises round cross-sections, which shank comprises a tapered shank core (8) and longitudinal ribs (10) which protrude laterally from the shank core (8) and which are joined to the shank core (8) as one piece,
**characterised in that**
- the longitudinal ribs (10) are multiply-stepped over their radial height and comprise cutting edges (12) on the steps which are thereby formed, and
- that the peripheries (11) of the longitudinal ribs (10) are formed by surface lines (7a) which are parallel to the longitudinal axis (7) of the shank (6),
- wherein each of the flanks of the longitudinal ribs (10) which originate from the shank core (8) is at the same distance from the longitudinal axis (7) over the entire length of the flanks, and
- each part between two adjacent steps which forms an external boundary of one of the longitudinal ribs (10) is at a constant distance from the longitudinal axis (7).

2. A femoral prosthesis according to claim 1,
**characterised in that**
the longitudinal ribs (10) are provided with grooves (13) for cuttings in the region of the steps.

3. A femoral prosthesis according to any one of the preceding claims,
**characterised in that**
the longitudinal ribs (10) are disposed regularly distributed over the periphery of the shank core (8).

4. A femoral prosthesis according to any one of the preceding claims,
**characterised in that**
the cross-section of each longitudinal rib (10) at the proximal end is an acute-angled triangle, a trapezium or a rectangle having a straight line as its base line which joins two points of the shank core and having the flanks which originate from these points as side lines.

5. A femoral prosthesis according to any one of the preceding claims,
**characterised in that**
it is made of round stock, wherein the longitudinal ribs (10) are formed by intermediate, worked-out grooves (14).

## Revendications

1. Prothèse fémorale comportant une tige (6) destinée à être enfoncée dans un fémur, qui est rétrécie vers l'extrémité distale (9) et dont la surface d'enveloppe présente des sections transversales rondes, laquelle tige comprend un noyau conique (8) ainsi que des nervures longitudinales (10) qui font saillie latéralement du noyau (8) de la tige et qui sont reliées d'un seul tenant à cette dernière,
caractérisée
- en ce que les nervures longitudinales (10) sont étagées plusieurs fois dans leur hauteur radiale et présentent des arêtes de coupe (12) sur les gradins ainsi formés et
- en ce que les surfaces de délimitation (11) des nervures longitudinales (10) sont constituées par des génératrices (7a), qui s'étendent parallèlement a l'axe longitudinal (7) de la tige (6),
- chacun des flancs des nervures longitudinales (10), partant du noyau (8) de la tige, présente, sur toute sa longueur totale, la même distance par rapport ä l'axe longitudinal (7), et
- chaque partie, formant une délimitation extérieure de l'une des nervures longitudinales (10) entre deux gradins voisins, présente une distance constante par rapport a l'axe longitudinal (7).

2. Prothèse fémorale selon la revendication 1, caractérisée
en ce que les nervures longitudinales (10) sont pourvues de rainures de serrage (13) dans la région des gradins.

3. Prothèse fémorale selon l'une des revendications précédentes, caractérisée
en ce que les nervures longitudinales (10) sont régulièrement réparties sur le pourtour du noyau (8) de la tige.

4. Prothèse fémorale selon l'une des revendications précédentes,
caractérisée
en ce que la section transversale de chaque nervure longitudinale (10) à l'extrémité proximale est un triangle à angle aigu, un trapèze ou un rectangle avec une droite reliant deux points du noyau de la tige, servant de ligne de base, et avec les flancs partant de ces points qui servent de lignes des côtés.

5. Prothèse fémorale selon l'une des revendications précédentes,
caractérisée
en ce qu'elle est fabriquée dans un matériau rond, les nervures longitudinales (10) étant formées par des rainures (14) intercalées, pratiquées dans la masse.
